(19) Europäisches Patentamt
European Patent Office
Office européen des brevets

(11) Veröffentlichungsnummer: **0 082 299 A1**

(12) **EUROPÄISCHE PATENTANMELDUNG**

(21) Anmeldenummer: 82110488.2

(22) Anmeldetag: 13.11.82

(51) Int. Cl.³: C 07 C 135/02
C 11 D 1/75

(30) Priorität: 19.11.81 DE 3145735

(43) Veröffentlichungstag der Anmeldung: 29.06.83 Patentblatt 83/26

(84) Benannte Vertragsstaaten: AT BE CH DE FR GB IT LI NL SE

(71) Anmelder: HOECHST AKTIENGESELLSCHAFT
Postfach 80 03 20
D-6230 Frankfurt/Main 80(DE)

(72) Erfinder: Blaschke, Günter, Dr.
Bajuwarenstrasse 36
D-8261 Winhöring(DE)

(72) Erfinder: Reng, Alwin
Im Schulzehnten 22
D-6233 Kelkheim (Taunus)(DE)

(72) Erfinder: Quack, Jochen Meinhard, Dr.
Wilhelm-Reuter-Strasse 16
D-6239 Eppstein/Taunus(DE)

(54) **Triamin-trioxide, Verfahren zu deren Herstellung und diese enthaltende Reinigungsmittel.**

(57) Triamin-trioxide der Formel

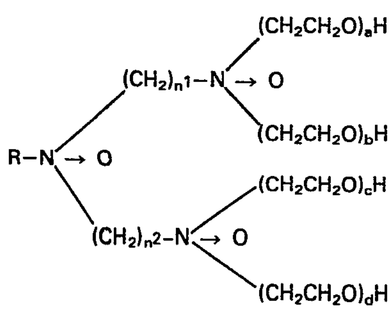

worin R einen gesättigten oder einen olefinisch ungesättigten Kohlenwasserstoffrest mit 1 bis 3 Doppelbindungen und von 8 bis 22 C-Atomen bedeutet,

$n^1$ und $n^2$ eine ganze Zahl von 2 bis 3, wobei $n^1$ und $n^2$ gleich oder verschieden sein können, darstellt, sowie

a, b, c und d, gleich oder verschieden, jeweils eine Zahl von 1 bis 5 ist, wobei die Summe a + b + c + d höchstens 10 betragen soll.

Die Verbindungen werden hergestellt aus primären Aminen der Formel $RNH_2$ durch Dicyanalkylierung, Hydrierung, Oxethylierung und Oxidation mit Wasserstoffperoxid. Sie eignen sich für die Formulierung von technischen, aber auch von kosmetischen Reinigungsmitteln.

EP 0 082 299 A1

Croydon Printing Company Ltd

0082299



HOECHST AKTIENGESELLSCHAFT HOE 81/F 928 Dr.SC-ei

Triamin-trioxide, Verfahren zu deren Herstellung und diese enthaltende Reinigungsmittel

Die Aufgabe von kosmetischen und technischen Reinigungsmitteln ist die Entfernung der verschiedensten Verunreinigungen von Oberflächen organischer oder anorganischer Natur. Die Topographie der zu reinigenden Oberflächen ist sehr unterschiedlich und hierdurch wird auch eine verschieden große Haftung der Verunreinigungen, wie Pigmente, Öle, Sebum, Staub, verursacht.

Bei der Reinigung solcher verschmutzter Oberflächen mit wäßrigen Tensidlösungen wird neben der erforderlichen Reinigungswirkung auch ein spezifisches, quantitatives und qualitatives Schaumbild während des Reinigungsprozesses verlangt. Die Art und Menge des Schaumes ist dabei für die meisten Reinigungsprozesse ein wichtiger Indikator für den zeitlichen Ablauf des Reinigungsvorganges. Vor allem bei den kosmetischen Reinigungspräparaten, wie Zahnpasten, Schaumbäder, Duschbäder, Shampoos und Seifen, verlangt der Konsument ein für den jeweiligen Reinigungsvorgang spezifisches Schaumbild. Bevorzugt wird dabei in den meisten Fällen ein möglichst feinblasiger, cremiger Schaum, der gleichzeitig eine Reihe von Vorteilen bei der Anwendung dieser kosmetischen Formulierungen bietet. Beispielsweise wirkt eine feinblasige und dichte sogenannte Schaumdecke bei der Benutzung von Schaumbädern wärmeisolierend, das heißt, das Badewasser kühlt wesentlich langsamer ab als das entsprechende schaumfreie Badewasser. Bei der Anwendung von Haarshampoos hingegen verhindert eine möglichst feinblasige Schaumstruktur

eine mechanische Schädigung durch zu starkes Zerren oder Reiben der Kopfhaare; auch bei Zahnpasten wird zum Schutz des Zahnfleisches während des Zähneputzens ein möglichst dichtes Schaumbild gewünscht. Ähnliches gilt auch für die Anwendung von schaumförmigen Dauerwellpräparaten. Schließlich wünscht der Verbraucher bei der Anwendung von Körperreinigungsmitteln, wie Duschbädern oder Haarshampoos, ein möglichst schnelles "Anspringen" des Schaumes, das heißt bei einer bestimmten mechanischen Belastung der Tensidlösung, beispielsweise beim Verreiben mit den Fingern, soll das maximale Schaumvolumen in möglichst kurzer Zeit erreicht werden.

Die meisten der praxisüblichen Tenside, wie Alkylsulfate, Alkylethersulfate, Alkylbenzolsulfonate, sekundäre Alkansulfonate, Fettalkoholpolyglykolether und Alkylphenolpolyglykolether, geben bei ihrer Anwendung in wäßriger Lösung einen relativ grobblasigen Schaum, so daß oft neben diesen Tensiden und Wasser eine dritte Komponente als sogenannter "Schaumbooster" zur Stabilisierung des Schaumes erforderlich ist, da meist mit zunehmender Teilchengröße des Schaumes auch ein zunehmender Zerfall in Abhängigkeit von der Zeit parallel geht.

Ein weiter Nachteil solcher handelsüblicher Tenside ist ihre beschränkte Einsatzmöglichkeit für die Herstellung von technischen Reinigungsmitteln, wie Kaltreinigern, Allzweckreinigern, Toilettenreinigern oder Geschirrspülmitteln, aus Gründen ihrer physikalischen oder chemischen Stabilität. So können beispielsweise Alkylsulfate, Alkylethersulfate oder Sulfobernsteinsäurehalbester nur in neutralem oder alkalischem Milieu eingesetzt werden, da im sauren pH-Bereich eine hydrolytische Spaltung unter Bildung von Schwefelsäure erfolgt, die zum Verlust der grenzflächenaktiven Eigenschaften führt.

Nicht-ionische Tenside, wie Alkylpolyglykolether oder Alkylarylpolyglykolether, werden hingegen im stark alkalischen Bereich zersetzt. Ein weiterer Nachteil vieler Tenside ist ferner ihre mangelnde Stabilität in Gegenwart wäßriger Elektrolytlösungen, da in Anwesenheit von Silicaten, Phosphaten oder Chloriden ein physikalischer Aussalzeffekt erfolgt. Andere Tenside wiederum werden in Gegenwart von Oxidationsmitteln abgebaut.

Es besteht daher ein Bedürfnis nach tensidischen Substanzen, die die vorgenannten Nachteile nicht aufweisen. Als tensidische Substanzen, die diese Nachteile nicht besitzen sollen, sind Aminoxide und Diamin-dioxide bekannt (vgl. M.J. Schick "Nonionic Surfactants", Marcel Dekker, New York, 1967, Seite 403 ff.; US-PS 31 97 509, US-PS 32 34 139), auch solche mit Hydroxyethyl-Substituenten. Jedoch können diese Substanzen das Bedürfnis nur mit Einschränkung erfüllen, denn sie weisen eine mangelhafte Hydrophilie und ein unbefriedigendes antistatisches Verhalten auf.

Erfindungsgemäß werden nunmehr dafür Triamin-trioxide der allgemeinen Formel

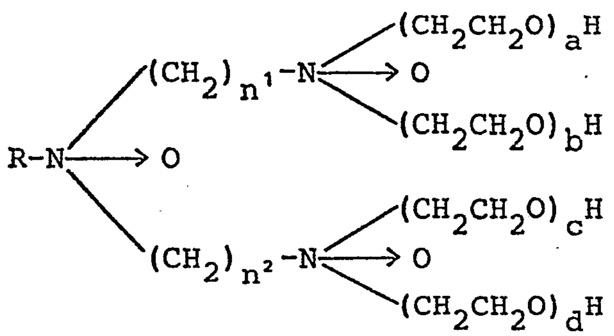

(I)

zur Verfügung gestellt, worin
R einen gesättigten oder einen olefinisch ungesättigten Kohlenwasserstoffrest mit 1 bis 3 Doppelbindungen und von 8 bis 22 C-Atomen bedeutet,
$n^1$ und $n^2$ eine ganze Zahl von 2 bis 3, wobei $n^1$ und $n^2$ gleich oder verschieden sein können, darstellt,
sowie a, b, c und d, gleich oder verschieden, jeweils eine Zahl von 1 bis 5 ist, wobei die Summe a + b + c + d höchstens 10 betragen soll.

In diesen erfindungsgemäßen Triamin-trioxiden der Formel I besitzt der Rest R 8 bis 22 C-Atome, er kann gesättigt oder ungesättigt mit 1 bis 3 olefinischen Doppelbindungen und er kann geradkettig oder verzweigt sein. Diese Alkyl- oder Alkenylreste, die dem primären Ausgangsamin bei der Herstellung der erfindungsgemäßen Triamin-trioxide entstammen, sind häufig Gemische oder Kettenschnitte, bevorzugt mit der Kettenverteilung der Reste von natürlichen Fettsäuren, wie insbesondere der Kokos-, Talg- oder Palmkernfettsäure, aus denen diese Ausgangsamine über den Weg der Nitrilhydrierung oder der Ammonolyse der entsprechenden Alkohole gewonnen werden. Die zur Herstellung der primären Amine mittels Ammonolyse verwendeten Alkohole können neben Fettalkoholen auch solche mit gerader oder verzweigter Kette aus dem Ziegler-Prozeß (Ethylenaufbaualkohole) oder aus der Oxosynthese sein.

Zur Herstellung der erfindungsgemäßen Verbindungen wird zunächst ein solches primäres Amin der Formel $RNH_2$ (II), wobei R die vorgenannte Bedeutung hat, mit 2 mol mindestens eines reaktionsfähigen Nitrils von 2 bis 3 C-Atomen (einschließlich der CN-Gruppe) zu einer Verbindung der allgemeinen Formel

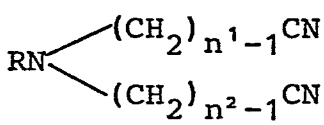

(III)

in einer Dicyanalkylierungsreaktion umgesetzt. Diese Reaktion ist bekannt, beispielsweise aus der US-PS 3 028 415. Sie kann sowohl mit saurer als auch mit basischer Katalyse, mit Hilfe von Lösungsmitteln, wie Wasser oder auch niederkettigen Alkoholen, drucklos oder unter erhöhtem Druck, kontinuierlich oder diskontinuierlich durchgeführt werden. Als saure Katalysatoren werden Essigsäure, Phosphorsäure, Salzsäure und

andere Mineralsäuren genannt (US-PS 3 615 797, US-PS 3 028 415, DE-OS 1 941 913), als basische Katalysatoren sind empfohlen worden Natrium- oder Kaliumhydroxid, Alkalialkoholate, Trimethylbenzylammoniumhydroxid und Morpholin (Kirk-Othmer, Encyclopedia of Chemical Technology, 1965, Band 6, Seite 634 ff.; H. A. Bruson "Cyanoethylation", Organic Reactions 5, 1949, Seite 79 ff., Verlag John Wiley and Sons, New York). Als Co-Katalysatoren oder auch als Lösungsvermittler werden Wasser oder niedere Alkohole, wie Methanol, Ethanol, Isopropanol oder Gemische derselben in Anteilen von 1 bis 20 Gew.-% zugegeben. Die Dicyanalkylierung wird unter Normaldruck oder leichtem bis mittlerem Überdruck von 1 bis 20 bar, gegebenenfalls in Gegenwart eines Inertgases, und bei Temperaturen von 60 bis 150 °C durchgeführt. Das Cyanalkylierungsmittel, vorzugsweise Acrylnitril oder Chloracetonitril, wird stöchiometrisch oder in einem bis zu vierfachen Überschuß angewandt.

Anschließend wird das so gewonnene Dicyanalkylierungsprodukt (III) in Gegenwart von Wasserstoff zu einer Verbindung der Formel

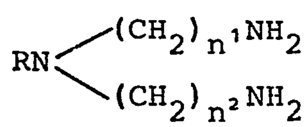

(IV)

reduziert und anschließend mit Ethylenoxid zu einer Verbindung der Formel

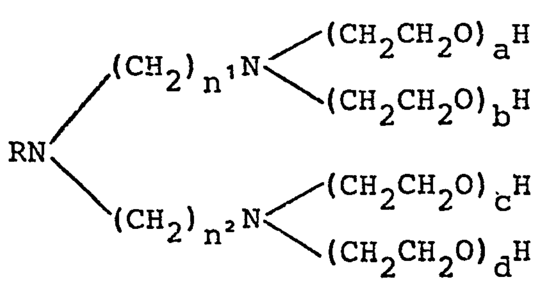

(V)

kondensiert. Beide Reaktionen sind für die in Rede stehenden Verbindungen ebenfalls bekannt (vgl. die oben bereits genannte US-PS 3 615 797). Die Reduktion

wird mit Raney-Nickel oder Raney-Kobalt oder aber mit Nickel- oder Kobalt-Trägerkatalysatoren durchgeführt, und zwar unter Einsatz von 1 bis 10 Gew.-% Katalysator, bevorzugt 1 bis 5 Gew.-%, und bei Drücken von 50 bis 200 bar Wasserstoff und Temperaturen von 60 bis 150 °C; die Reaktionszeit beträgt dabei etwa 1 bis 5 Stunden.

Die Oxethylierungsreaktion wird in Druckgefäßen durchgeführt, und zwar bei erhöhter Temperatur im Bereich von 110 bis 170 °C und erhöhtem Druck von 1 bis 5 bar. Ein Katalysator ist nicht erforderlich, wenn vorzugsweise nur eine Ethylenoxid-Einheit pro Kette angelagert werden soll. Wird ein Katalysator eingesetzt, so ergeben sich vorzugsweise Ethylenoxidketten, die mehr als eine Einheit enthalten. Es werden 4 bis 10 mol Ethylenoxid pro 1 mol der Verbindung IV in der Reaktion eingesetzt, vorzugsweise 4 bis 5 mol. Das Ethylenoxid kann dabei mit einem Inertgas verdünnt werden.

Danach wird das so erhaltene Ethylenoxid-Anlagerungsprodukt in an sich bekannter Weise zu dem erfindungsgemäßen Triamin-trioxid der Formel

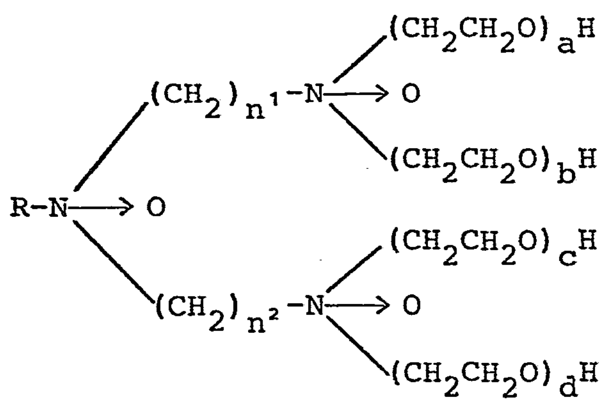

(I)

umgesetzt, und zwar in wäßriger Lösung mit 30 oder 70 gew.-%igem Wasserstoffperoxid. Die Reaktion wird bei 50 bis 90 °C mit 5 bis 10 %igem Überschuß, bezogen auf die erforderliche Menge von 3 mol, an Wasserstoffperoxid durchgeführt.

Vorteilhafterweise stellt man durch geeignete Wahl des Wassergehaltes bei der letzten Reaktionsstufe die erfindungsgemäßen Triamin-trioxide der Formel I als 30 bis 40 gew.-%ige wäßrige Einstellungen her.

Die erfindungsgemäßen Triamin-trioxide eignen sich besonders zur Herstellung von chemisch und physikalisch stabilen, technischen und kosmetischen Reinigungsmitteln. Die besonderen Charakteristika der mit diesen Triamin-trioxiden hergestellten Reinigungspräparate sind feinblasiger Schaum, gutes Anschäumvermögen und Stabilität in einem weiten pH-Bereich.
Weitere Vorteile sind die physikalische Stabilität in Gegenwart von Elektrolyten, wie Silicaten, Phosphaten, Bromaten und dergleichen, das heißt, es treten in wäßriger Verdünnung keine Trübungen oder Ausfällungen auf, wie sie bei vielen handelsüblichen Tensiden vorkommen. Auch in Gegenwart von Oxidationsmitteln, wie Wasserstoffperoxid oder Chlorbleichlauge, tritt keine chemische Veränderung ein.

Die erfindungsgemäßen Triamin-trioxide sind insbesondere zur Formulierung von technischen Reinigungsmitteln, also Schaumreinigern für textile Flächen wie Teppichreiniger, oder insbesondere Reinigungsmitteln für harte Oberflächen, wie beispielsweise Geschirr- und Flaschenspülmittel, Fußbodenreiniger, Sanitärreiniger oder sogenannte Allzweckreinigungsmittel, geeignet.

Die erfindungsgemäßen Triamin-trioxide, wie sie in der obengenannten Formel I definiert sind, sind ferner geeignet zum Einsatz in kosmetischen Reinigungsmitteln, das heißt Körperreinigungsmitteln wie Schaumbädern, Duschbädern, Fuß- und Handwaschmitteln oder Intimwaschmitteln sowie ferner in Haarwaschmitteln. Beim Einsatz

in Körperreinigungsmitteln wird eine deutliche Verbesserung des Hautgefühls nach der Anwendung erzielt, beim Einsatz in Shampoos wird eine Verbesserung der Kämmbarkeit sowohl der trockenen als auch der nassen Haare erreicht bei gleichzeitig weichmachender Wirkung, die sich in einem angenehmen Haargriff bemerkbar macht.

Die erfindungsgemäßen Triamin-trioxide können in solchen flüssigen, pulverförmigen oder auch aerosolförmigen technischen und kosmetischen Reinigungsmitteln sowohl allein als auch in Kombination mit den üblicherweise in solchen Mitteln eingesetzten anionischen, kationischen, nicht-ionischen und amphoteren Tensiden verwendet werden. Dafür geeignete anionische Tenside sind beispielsweise Seife, Fettalkoholsulfate, Alkylethersulfate, Fettsäurekondensationsprodukte wie Tauride, Methyltauride, Sarkoside, ferner α-Olefinsulfonate, Hydroxyalkansulfonate, sekundäre Alkansulfonate, Amidethersulfate oder Alkylbenzolsulfonate. Als nicht-ionische Tenside können beispielsweise Polyglykolmonoalkylether und -monoester, Aminoxide und Ethylenoxid-Propylenoxid-Kondensationsprodukte verwendet werden. Daneben ist auch die Kombination mit anderen amphoteren Tensiden wie Alkyl-Betainen, Alkylamido-Betainen, Imidazolinderivaten oder Sulfo-Betainen möglich. Schließlich können die erfindungsgemäßen Triamin-trioxide auch in Abmischung mit kationischen Tensiden wie Cetyltrimethylammoniumchlorid, Cetyltrimethylammoniumbromid, Pentaoxyethylstearylammoniumchlorid, quaternierten Etheraminen oder polymeren quartären Ammoniumverbindungen eingesetzt werden. Weitere Zusätze, die auf sonst übliche Weise in kosmetischen Reinigungsmitteln verwendet werden, können mit den Triamin-trioxiden kombiniert werden. Dies sind beispielsweise viskositätserhöhende oder -erniedrigende Verbindungen wie Celluloseether, Elektrolyte, wie beispielsweise Natriumchlorid

oder Ammoniumchlorid, Fettsäurepolyglykolester, Alkanolamide, Magnesium-Aluminium-Silicate, Polyglykole, Glycerin und Ethanol. Ferner kommen als solche Zusätze in Frage Parfümöle und spezielle Riechstoffe, Antiseptika, schuppenentfernende oder pilztötende Mittel, Überfettungsmittel, Konservierungsmittel, Farbstoffe und Perlglanz gebende Substanzen. Bei der Verarbeitung zu pulverförmigen Präparaten können weiterhin üblicherweise benutzte Füll- und Trägersubstanzen wie hochdisperse, amorphe Kieselsäure, Natriumsulfat, Magnesium-Aluminium-Silicat, Stärkederivate und dergleichen verwendet werden. Schließlich können auch im Falle von aerosolförmigen Präparaten übliche Treibgase beigemischt werden. Die Regulierung des gewünschten pH-Wertes kann mit anorganischen oder organischen Säuren oder Alkalien vorgenommen werden. In technischen Reinigungsmitteln können als übliche Hilfsmittel Chelatbildner und gegebenenfalls auch Kunststoffdispersionen zugesetzt werden. Ferner sind übliche Zusätze hier Bleichmittel, Chlorabspalter oder andere Desinfektionsmittel. Zur Verbesserung der Abrasionswirkung eignen sich Kreide, hochdisperse amorphe Kieselsäure, Phosphate und Kunststoffe. Zur Verbesserung der Fett- und Schmutzlöseeigenschaften können auch Lösungsmittel wie Testbenzin oder Isopropylalkohol oder andere reinigungsverstärkende Mittel zugefügt werden. Schließlich eignen sich die erfindungsgemäßen Triamin-trioxide als Textilwaschmittel.

Der Gehalt an den erfindungsgemäßen Triamin-trioxiden in solchen Formulierungen beträgt üblicherweise 0,5 bis 40 Gew.-%.

Die folgenden Beispiele sollen die Erfindung näher erläutern:

## Herstellungsbeispiele

### Beispiel 1

In einem 2-l-Vierhalskolben mit Rückfluß, Thermometer, Rührer und Dosiergefäß werden 670 g Cocosfettamin (Zusammensetzung in Mol-% bezüglich der Reste R: $C_8$ 6 %, $C_{10}$ 6 %, $C_{12}$ 54 %, $C_{14}$ 18 %, $C_{16}$ 8 %, $C_{18}$ 8 %), 68 g Wasser, 34 g Methanol und 14 g konzentrierte Essigsäure auf 60 °C erhitzt. Innerhalb einer Stunde tropft man 373 g Acrylnitril zu und rührt weitere 24 bis 36 h bei 75 °C unter Rückfluß. Anschließend wird mit 13 g NaOH und 120 g Wasser neutralisiert, das Waschwasser separiert und das Produkt im Vakuum vom Restwasser und Lösungsmittel befreit. Man erhält 1000 g Cocosfettamino-di-propionitril (Ausbeute 95,9 %).

Ein 5-l-Autoklav wird mit 2020 g Cocosfettamino-di-propionitril, 3 g Kobalt-Trägerkontakt (Träger: Kieselgur) und 300 ml flüssigen Ammoniaks beschickt. Die Hydrierung erfolgt bei 150 bis 180 bar $H_2$ und 110 bis 140 °C innerhalb von 3 Stunden. Nach Abfiltrieren des Katalysators erhält man 2010 g Produkt, das 85 bis 95 % Bis(3-aminopropyl)-cocosfettamin enthält.

In einem 2-l-Druckgefäß mit Thermometer, Rührer und Ethylenoxid-Einlaß und -Auslaß werden 954 g Bis(3-aminopropyl)-cocosfettamin auf 130 °C unter Rühren aufgeheizt. 667 g Ethylenoxid werden bei einem Druck von 1 bis 3 bar aufgegeben. Die Gewichtszunahme nach 3 Stunden Reaktionszeit entspricht einem Kondensationsprodukt des Triamins mit 4 bis 5 mol Ethylenoxid. Man erhält 1605 g dieses Oxethylats (99 %).

261 g Bis(3-aminopropyl)-cocosfettaminoxethylat und 559 g Wasser werden in einem 2-l-Reaktionsgefäß vorgelegt und unter Rühren auf 60 °C erwärmt. Bei dieser Temperatur werden innerhalb 1 h 146 g 70 gew.-%iges Wasserstoffperoxid zugegeben, danach wird noch 12 h bei 60 °C nachgerührt. Man erhält das entsprechende Triamin-trioxid in 960 g einer 30 gew.-%igen wäßrigen Lösung.

Beispiel 2

670 g Laurylamin (Anteil $C_{12}$ 73 Mol-%, Anteil $C_{14}$ 23 Mol-%) werden in 68 g Wasser, 34 g Methanol und 14 g konzentrierter Essigsäure in der bereits in Beispiel 1 beschriebenen Weise mit 373 g Acrylnitril umgesetzt und dann hydriert.
Nach der Hydrierung erhält man 2020 g Bis(3-aminopropyl)-laurylamin. 954 g dieses Triamins werden mit 640 g Ethylenoxid kondensiert. Man erhält 1590 g an Bis(3-aminopropyl)-laurylaminoxethylat (99 %).
257 g dieses Oxethylats und 538 g Wasser werden mit 146 g 70 gew.-%igem $H_2O_2$ zur Reaktion gebracht. Man erhält das Triamin-trioxid in 935 g einer 30 gew.-%igen wäßrigen Lösung.

Beispiel 3

844 g Myristylamin werden in 68 g Wasser, 34 g Methanol und 14 g konzentrierter Essigsäure mit 373 g Acrylnitril bei 75 °C umgesetzt. Man erhält 1164 g Myristylamino-di-propionitril. 2052 g des Di-propionitrils werden wie in Beispiel 1 mit Kobalt-Katalysator hydriert. Man erhält 2045 g Bis(3-aminopropyl)-myristylamin. 1095 g dieses Triamins werden mit 647 g Ethylenoxid kondensiert. Man erhält 1725 g an Bis(3-aminopropyl)-myristylaminoxethylat (99 %). 290 g dieses Oxethylats und 615 g Wasser werden mit 146 g 70 gew.-%igem $H_2O_2$

zur Reaktion gebracht. Man erhält das Triamin-trioxid in 1050 g einer 30 gew.-%igen wäßrigen Lösung.

Beispiel 4

1056,0 g Octylamin werden in 106 g Wasser, 53 g Methanol und 21,1 g konzentrierter Essigsäure mit 849,6 g Acrylnitril bei 75 °C umgesetzt. Man erhält 1810 g Octylamino-di-propionitril. 1980 g des Di-propionitrils werden wie in Beispiel 1 mit Kobalt-Katalysator hydriert. Man erhält 1970 g Bis(3-aminopropyl)-octylamin. 998 g dieses Triamins werden mit 845 g Ethylenoxid kondensiert. Man erhält 1840 g an Bis(3-aminopropyl)-octylaminoxethylat (99 %). 230 g dieses Oxethylats und 475 g Wasser werden mit 146 g 70 gew.-%igem $H_2O_2$ zur Reaktion gebracht. Man erhält das Triamin-trioxid in 850 g einer 30 gew.-%igen wäßrigen Lösung.

Beispiel 5

929 g Talgfettamin werden in 68 g Wasser, 34 g Methanol und 14 g konzentrierter Essigsäure mit 373 g Acrylnitril bei 75 °C umgesetzt. Man erhält 1237 g Talgfettaminodi-propionitril, das gemäß Beispiel 1 zum entsprechenden Amin hydriert wird. Man erhält 1230 g Bis(3-aminopropyl)-talgfettamin.

Die Oxethylierungsreaktion wird in zwei Stufen durchgeführt. Zunächst werden 1154 g Bis(3-aminopropyl)-talgfettamin nach der Methode von Beispiel 1 mit 647 g (4,7 mol) Ethylenoxid umgesetzt. Unter Einsatz von üblichen 0,2 Gew.-%, bezogen auf das Amin, an wäßriger Natronlauge (50 %ig) wird anschließend mit weiteren 5,3 mol Ethylenoxid umgesetzt, so daß die gesamte Gewichtszunahme einem Kondensationsprodukt des Triamins

mit 10 mol Ethylenoxid entspricht. 300 g dieses Oxethylats und 639 g Wasser werden mit 146 g 70 gew.-%igem $H_2O_2$ umgesetzt. Man erhält das Triamin-trioxid in 1020 g einer 30 gew.-%igen wäßrigen Lösung.

Die analytischen Daten der erfindungsgemäßen Triamintrioxide und deren Vorprodukte sind in Tabelle I zusammengefaßt.

TABELLE I

| Beispiel/ (Alkylrest) | Alkylamino-di-propionitril | | Bis(3-aminopropyl)-alkyl-amin | | | | Oxethylat | | | Triamin-trioxid | |
|---|---|---|---|---|---|---|---|---|---|---|---|
| | AZ | tert. N (%) | AZ | prim. N (%) | sek. N (%) | tert. N (%) | AZ | tert. N (%) | $\Sigma a+b+c+d$ Äquiv. EO* pro mol | AZ | Gehalt Gew.-% |
| 1 (Cocos) | 34,3 | 94 | 97,3 | 66,2 | 3,3 | 30,5 | 57,5 | >98 | 4,9 | 15,8 | 29,5 |
| 2 (Lauryl) | 34,4 | 95 | 97,5 | 65,9 | 2,9 | 31,2 | 58,3 | >98 | 4,7 | 16,0 | 30,0 |
| 3 (Myristyl) | 29,2 | 94 | 82,2 | 65,5 | 3,5 | 31,0 | 51,7 | >98 | 4,9 | 14,0 | 29,4 |
| 4 (Octyl) | 42,7 | 94 | 120,2 | 65,1 | 3,0 | 31,9 | 65,1 | >98 | 4,8 | 17,5 | 29,7 |
| 5 (Talgfett) | 27,4 | 94 | 78,0 | 66,1 | 2,6 | 31,3 | 50,0 | >98 | 4,9 | 13,6 | 29,5 |

* EO = Ethylenoxid

0082299

Die obengenannten Daten werden wie folgt bestimmt:

Alkylamino-di-propionitril:

Die Bestimmung der Aminzahl (AZ) und des Gehaltes an tertiärem Stickstoff erfolgt durch Titration mit 0,1 N $HClO_4$ in Eisessig bzw. Essigsäureanhydrid. Die Aminzahl errechnet sich aus $AZ = \frac{\text{ml } 0{,}1 \text{ N } HClO_4}{\text{Einwaage in g}}$

Bis(3-aminopropyl)-alkylamin:

Die Bestimmung der Aminzahl und der Aminverteilung erfolgt durch Titration mit 0,2 N-isopropanolischer HCl in wasserfreiem Medium. Die Aminverteilung wird durch Blockierung des basischen Aminstickstoffs mit Salicylaldehyd (primäres N) bzw. Phenylisothiocyanat (primäres und sekundäres N) durchgeführt.

Oxethylat:

Die Bestimmung der Aminzahl und des Gehaltes an tertiärem Stickstoff erfolgt durch Titration mit 0,1 N $HClO_4$ in Eisessig bzw. Essigsäureanhydrid.
Die aufgenommenen Mole Ethylenoxid errechnen sich aus den Aminzahlen bzw. aus der Massenzunahme gegenüber der Vorstufe.

Triamin-trioxid:

Die Bestimmung der Aminzahl erfolgt durch Titration mit 0,2 N isopropanolischer HCl in wasserfreiem Medium. Die Bestimmung des Gehalts an Triamin-trioxid erfolgt durch Redoxtitration mit Titan(III)-chlorid/$NH_4Fe(SO_4)_2 \cdot 6H_2O$.

Die nachstehenden Anwendungsbeispiele veranschaulichen die Einsatzmöglichkeiten der Triamin-trioxide in technischen und kosmetischen Reinigungsmitteln auch in speziellen Präparaten, wie Rasierseifen, Dauerwellpräparaten oder anderen Produkten, wo neben den Schaumeigenschaften vor allem eine chemische und physikalische Stabilität verlangt wird.

Beispiel 1 A

Saures Reinigungsmittel

| | |
|---|---|
| Triamin-trioxid der Formel I, hergestellt nach Beispiel 1 | 5,0 % |
| Orthophosphorsäure | 30,0 % |
| Wasser | ad 100,0 % |

Beispiel 2 A

Desinfektionsreiniger

| | |
|---|---|
| Triamin-trioxid der Formel I, hergestellt nach Beispiel 2 | 5,0 % |
| Lauryldimethylbenzylammoniumchlorid | 5,0 % |
| Citronensäure | 0,2 % |
| Wasser | ad 100,0 % |

Beispiel 3 A

Synthetische Seife

| | |
|---|---|
| Triamin-trioxid der Formel I, hergestellt nach Beispiel 1 | 5,0 % |
| Cocosisothionat-Natriumsalz | 60,0 % |
| Stearinsäure | 10,0 % |
| Titandioxid | 2,0 % |
| Cetylalkohol | ad 100,0 % |

Beispiel 4 A

Universalwaschpaste

| | |
|---|---|
| Triamin-trioxid der Formel I, hergestellt nach Beispiel 1 | 10,0 % |
| sekundäres Alkansulfonat-Natriumsalz ($C_{13}$-$C_{17}$) | 15,0 % |
| Natriumtripolyphosphat | 3,0 % |
| Natriumchlorid | 5,0 % |
| Carboxymethylcellulose | 5,0 % |
| Wasser, Konservierungsmittel | ad 100,0 % |

Beispiel 5 A

Allzweckreiniger

| | |
|---|---|
| Triamin-trioxid der Formel I, hergestellt nach Beispiel 1 | 10,0 % |
| Nonylphenol, kondensiert mit 10 mol Ethylenoxid | 3,0 % |
| Cocosfettsäuremonoethanolamid, kondensiert mit 5 mol Ethylenoxid | 2,0 % |
| Tetrakaliumpyrophosphat | 6,0 % |
| Wasser, Farbstoff, Parfümöl, Konservierungsmittel | ad 100,0 % |

Beispiel 6 A

Fußbodenreiniger

| | |
|---|---|
| Triamin-trioxid der Formel I, hergestellt nach Beispiel 1 | 7,0 % |
| Natriumtripolyphosphat | 5,0 % |
| Kaliumhydroxid | 1,5 % |
| Wasser, Parfümöl | ad 100,0 % |

Beispiel 7 A

Grillreiniger

| | |
|---|---|
| Triamin-trioxid der Formel I, hergestellt nach Beispiel 1 | 15,0 % |
| Nonylphenol, kondensiert mit 6 mol Ethylenoxid | 8,0 % |
| Stearinsäure | 1,0 % |
| Natriumhydroxid | 10,0 % |
| Calciumcarbonat | 20,0 % |
| Wasser, Konservierungsmittel | ad 100,0 % |

Beispiel 8 A

Sanitärreiniger

| | |
|---|---|
| Triamin-trioxid der Formel I, hergestellt nach Beispiel 2 | 10,0 % |
| Dodecylbenzolsulfonat-Natriumsalz | 5,0 % |
| Isopropanol | 5,0 % |
| Orthophosphorsäure | 8,0 % |
| Wasser, Desinfektionsmittel | ad 100,0 % |

Beispiel 9 A

Toilettenreiniger

| | |
|---|---|
| Triamin-trioxid der Formel I, hergestellt nach Beispiel 1 | 5,0 % |
| α-Olefinsulfonat ($C_{14}$-$C_{16}$) | 2,0 % |
| Nonylphenol, kondensiert mit 8 mol Ethylenoxid | 2,0 % |
| Orthophosphorsäure | 10,0 % |
| Butylglykol | 5,0 % |
| Wasser | ad 100,0 % |

Beispiel 10 A

Feinwaschmittel

| | |
|---|---|
| Triamin-trioxid der Formel I, hergestellt nach Beispiel 2 | 10,0 % |
| Lauryltriglykolethersulfat-Natriumsalz | 2,0 % |
| Isotridecylalkohol, kondensiert mit 8 mol Ethylenoxid | 2,0 % |
| p-Toluolsulfonat | 4,0 % |
| Tetrakaliumpyrophosphat | 12,0 % |
| Natriumtripolyphosphat | 4,0 % |
| Wasser, Konservierungsmittel, Parfümöl | ad 100,0 % |

Beispiel 11 A

Hochdruckreiniger

| | |
|---|---|
| Triamin-trioxid der Formel I, hergestellt nach Beispiel 2 | 5,0 % |
| Octanphosphonsäure | 3,0 % |
| Natriumtripolyphosphat | 10,0 % |
| Kaliumhydroxid | 3,0 % |
| Natriummetasilicat · $5H_2O$ | 10,0 % |
| Ethylendiamin, kondensiert mit 30 mol Ethylenoxid und 60 mol Propylenoxid | 2,0 % |
| Wasser | ad 100,0 % |

Beispiel 12 A

Sanitärreiniger

| | |
|---|---|
| Triamin-trioxid der Formel I, hergestellt nach Beispiel 2 | 5,0 % |
| sekundäres Alkansulfonat-Natriumsalz (Alkanrest $C_{13}$-$C_{17}$) | 2,0 % |
| Natriumhydroxid | 0,5 % |
| Natriumhypochlorit-Lösung (150 g Aktivchlor/l) | 50,0 % |
| Wasser, Parfümöl | ad 100,0 % |

Beispiel 13 A

Flüssiges Waschmittel

| | |
|---|---|
| Triamin-trioxid der Formel I, hergestellt nach Beispiel 2 | 15,0 % |
| Kaliumseife (40 %ig) | 10,0 % |
| Octanphosphonsäure | 5,0 % |
| Kaliumhydroxid | 3,0 % |
| Kaliumtripolyphosphat (50 %ig) | 30,0 % |
| Natriummetasilicat | 5,0 % |
| Wasser, Konservierungsmittel, optische Aufheller, Parfümöl | ad 100,0 % |

Beispiel 14 A

Kreidereiniger

| | |
|---|---|
| Triamin-trioxid der Formel I, hergestellt nach Beispiel 2 | 8,0 % |
| Nonylphenol, kondensiert mit 8 mol Ethylenoxid | 1,0 % |
| Laurylalkohol | 1,5 % |
| Tetranatriumpyrophosphat | 3,0 % |
| Kreide | 40,0 % |
| Wasser, Parfümöl, Formalin | ad 100,0 % |

Beispiel 15 A

Haarshampoo

| | |
|---|---|
| Triamin-trioxid der Formel I, hergestellt nach Beispiel 1 | 15,0 % |
| Parfümöl | 0,2 % |
| Citronensäure | 0,3 % |
| Wasser, Konservierungsmittel | ad 100,0 % |

Beispiel 16 A

Haarshampoo für fettige Haare

| | |
|---|---|
| Triamin-trioxid der Formel I, hergestellt nach Beispiel 2 | 5,0 % |
| Lauryldiglykolethersulfat-Natriumsalz | 10,0 % |
| Parfümöl | 0,1 % |
| Wasser, Konservierungsmittel | ad 100,0 % |

Beispiel 17 A

Duschbad

| | |
|---|---|
| Triamin-trioxid der Formel I, hergestellt nach Beispiel 1 | 10,0 % |
| Cocostriglykolethersulfosuccinat-Dinatriumsalz | 5,0 % |
| Cocosfettsäuremonoethanolamid | 1,0 % |
| Wasser, Konservierungsmittel, Parfümöl | ad 100,0 % |

Beispiel 18 A

Schaumbad

| | |
|---|---|
| Triamin-trioxid der Formel I, hergestellt nach Beispiel 2 | 20,0 % |
| Laurylsulfat-Triethanolaminsalz | 5,0 % |
| sekundäres Alkansulfonat-Natriumsalz ($C_{13}$-$C_{17}$) | 5,0 % |
| Ölsäureethanolamid | 2,0 % |
| Hydroxyethylcelluloseether | 1,3 % |
| Wasser, Konservierungsmittel, Parfümöl | ad 100,0 % |

Beispiel 19 A

Babyshampoo

| | |
|---|---|
| Triamin-trioxid der Formel I, hergestellt nach Beispiel 2 | 10,0 % |
| Lauroylsarkosid-Natriumsalz | 3,0 % |
| Citronensäure | 0,2 % |
| Wasser, Konservierungsmittel, Farbstoff, Konsistenzgeber, Parfümöl | ad 100,0 % |

Beispiel 20 A

Handwaschmittel

| | |
|---|---|
| Triamin-trioxid der Formel I, hergestellt nach Beispiel 1 | 10,0 % |
| α-Olefinsulfonat ($C_{14}$-$C_{16}$) | 3,0 % |
| Cocosfettsäurediethanolamid | 2,0 % |
| Triethylenglykoldistearat | 1,0 % |
| Wasser, Konsistenzgeber, Konservierungsmittel, Farbstoff | ad 100,0 % |

Beispiel 21 A

Konditioniershampoo

| | |
|---|---|
| Triamin-trioxid der Formel I, hergestellt nach Beispiel 1 | 10,0 % |
| Cocosbetain | 3,0 % |
| Cetyltrimethylammoniumchlorid | 1,0 % |
| Citronensäure | 0,2 % |
| Wasser, Konsistenzgeber, Farbstoff | ad 100,0 % |

Beispiel 22 A

Antischuppenshampoo

| | |
|---|---|
| Triamin-trioxid der Formel I, hergestellt nach Beispiel 1 | 12,0 % |
| Cocosfettsäuremethyltaurid-Natriumsalz | 5,0 % |
| Trioctaglykolether-o-phosphorsäureester | 1,0 % |
| Zinkpyrithion | 1,0 % |
| Wasser, Konservierungsmittel, Konsistenzgeber, Farbstoff | ad 100,0 % |

Beispiel 23 A

Haarglättungsmittel

| | |
|---|---|
| Triamin-trioxid der Formel I, hergestellt nach Beispiel 2 | 1,0 % |

| | |
|---|---|
| Natriumhydroxid | 2,0 % |
| Tragant | 2,0 % |
| Cetylalkohol | 3,0 % |
| Wasser | ad 100,0 % |

Beispiel 24 A

Haarkurmittel

| | |
|---|---|
| Triamin-trioxid der Formel I, hergestellt nach Beispiel 1 | 1,0 % |
| Di-stearyldimethylammoniumchlorid | 3,0 % |
| Stearylpentaoxethylammoniumchlorid | 1,0 % |
| Cetylstearylalkohol | 3,0 % |
| Wasser, Farbstoff, Parfümöl | ad 100,0 % |

Beispiel 25 A

Haarfärbemittel

| | |
|---|---|
| Triamin-trioxid der Formel I, hergestellt nach Beispiel 2 | 0,5 % |
| p-Toluidin-diamin | 1,0 % |
| Resorcin | 1,0 % |
| Cetylstearylalkohol | 8,0 % |
| Ammoniak | 1,0 % |
| Natriumsulfit | 0,1 % |
| Wasser | ad 100,0 % |

Beispiel 26 A

Dauerwellfixiermittel

| | |
|---|---|
| Triamin-trioxid der Formel I, hergestellt nach Beispiel 2 | 2,0 % |
| Wasserstoffperoxid | 2,0 % |
| Citronensäure | 0,4 % |
| Wasser, Parfümöl, Stabilisator | ad 100,0 % |

Beispiel 27 A

Rasierseife

| | |
|---|---|
| Triamin-trioxid der Formel I, hergestellt nach Beispiel 1 | 3,0 % |
| Myristinsäure | 10,0 % |
| Stearinsäure | 23,0 % |
| Cocosfettsäure | 6,0 % |
| Kaliumhydroxid | 7,0 % |
| Natriumhydroxid | 0,5 % |
| Triethanolamin | 1,0 % |
| Wasser, Parfümöl | ad 100,0 % |

Beispiel 28 A

Zahnpaste

| | |
|---|---|
| Triamin-trioxid der Formel I, hergestellt nach Beispiel 2 | 1,5 % |
| Dicalciumphosphat | 35,0 % |
| Calciumcarbonat | 10,0 % |
| Glycerin | 5,0 % |
| Hydroxyethylcelluloseether | 2,0 % |
| Saccharin | 0,1 % |
| Natrium-fluormonophosphat | 0,76 % |
| Wasser, Aromaöl, Konservierungsmittel | ad 100,0 % |

Beispiel 29 A

Geschirrspülmittel

| | |
|---|---|
| Triamin-trioxid der Formel I, hergestellt nach Beispiel 1 | 10,0 % |
| sekundäres Alkansulfonat-Natriumsalz (Alkanrest $C_{13}$ - $C_{17}$) | 20,0 % |
| Natriumtripolyphosphat | 1,0 % |
| Ethanol | 3,0 % |
| Wasser | ad 100,0 % |

0082299

Die ausgezeichnete Schaumstabilität, das heißt die Beständigkeit des Schaumes in Abhängigkeit von der Zeit, zeigt Tabelle Ia und Ib in einem Vergleich der erfindungsgemäßen Triamin-trioxide mit Laurylsulfat-Natriumsalz (Ia) und einem sekundären Alkansulfonat (Kettenschnitt $C_{13}$ - $C_{17}$) (Ib) beziehungsweise deren Mischung mit der erfindungsgemäßen Verbindung im Verhältnis 1 : 1. Die Bestimmung des Schaumverhaltens erfolgte nach dem Lochscheibenschlagverfahren gemäß DIN-Norm 53 902; hierbei wird der Schaum durch Schlagen einer wäßrigen Tensidlösung in einem graduierten Meßzylinder mit Hilfe einer perforierten Platte erzeugt und die Schaumhöhen in bestimmten Zeitintervallen visuell abgelesen.

## Tabelle Ia

Schaumhöhe in ml
Konzentration 0,2 % Wirkstoff
Temperatur 40 °C
Wasserhärte 20 ° dH

| Zeit | Laurylsulfat-Natriumsalz | Triamin-trioxid von Herst.Beispiel 1 | Mischung 1 : 1 |
|---|---|---|---|
| sofort | 400 | 700 | 570 |
| 5 min | 360 | 680 | 560 |
| 10 min | 300 | 630 | 530 |
| 15 min | 280 | 600 | 510 |
| 20 min | 200 | 480 | 500 |
| 25 min | 140 | 370 | 480 |
| 30 min | 80 | 270 | 460 |

Tabelle Ib

| Zeit | sek. Alkansulfonat | Triamin-trioxid von Herst.Beispiel 1 | Mischung 1 : 1 |
|---|---|---|---|
| sofort | 310 | 700 | 580 |
| 5 min | 280 | 680 | 550 |
| 10 min | 210 | 630 | 510 |
| 15 min | 200 | 600 | 420 |
| 20 min | 170 | 480 | 400 |
| 25 min | 120 | 370 | 380 |
| 30 min | 80 | 270 | 250 |

Um das sogenannte Anschäumvermögen sowie auch das Wasserbindevermögen der Schäume festzustellen, wurde die Reibschaummethode nach Wilmsmann, Fette-Seifen-Anstrichmittel 66, 955 (1964), verwendet. Dabei wird der Schaum mit Hilfe einer rotierenden Kunststoffbürste 5 Minuten lang erzeugt und anschließend die Schaumwasserabscheidung ebenfalls innerhalb von 5 Minuten ermittelt. Die entsprechenden Ergebnisse sind in Tabelle II dargestellt, und zwar im Vergleich mit Laurylsulfat-Natriumsalz.

Tabelle II

Schaumvolumen in ml

| | |
|---|---|
| Konzentration | 0,2 % Wirkstoff |
| Temperatur | 40 °C |
| Wasserhärte | 20 ° dH |

Tabelle II (fortgesetzt)

| | Zeit | Triamin-trioxid von Herst.Beispiel 1 | Laurylsulfat-Natriumsalz |
|---|---|---|---|
| Schaum-ent-wick-lung | 30 sec | 1200 | 1150 |
| | 1 min | 1230 | 1170 |
| | 2 min | 1330 | 1250 |
| | 3 min | 1410 | 1300 |
| | 4 min | 1470 | 1360 |
| | 5 min | 1500 | 1430 |
| Schaum-beständig-keit | 0 min | 1500 | 1430 |
| | 1 min | 1400 | 1350 |
| | 2 min | 1340 | 1280 |
| | 3 min | 1280 | 1250 |
| | 4 min | 1250 | 1170 |
| | 5 min | 1200 | 1100 |

Ein weiterer Vorteil der erfindungsgemäßen Triamin-trioxide ist die Stabilität auch im sauren pH-Bereich; die Messung entsprechender Werte erfolgte durch 10-wöchige Lagerung der Substanzen in 20 gew.-%iger Konzentration in Wasser bei +40 °C und regelmäßiger Messung des pH-Wertes. Tabelle III zeigt, daß das erfindungsgemäße Triamin-trioxid im Gegensatz zu dem hydrolyseempfindlichen Alkylethersulfat keinen Abfall des pH-Wertes aufweist.

## T a b e l l e  III

pH-Stabilität 40 °C

| Zeit | Triamin-trioxid von Herst.Beispiel 1 | Lauryldiglykolethersulfat-Natriumsalz |
|---|---|---|
| sofort | 7,7 | 7,6 |
| 1 Woche | 7,7 | 7,0 |
| 2 Wochen | 7,8 | 5,8 |
| 3 Wochen | 7,9 | 4,6 |
| 4 Wochen | 8,0 | 4,0 |
| 5 Wochen | 8,0 | 3,7 |
| 6 Wochen | 8,0 | 3,5 |
| 7 Wochen | 8,0 | 3,4 |
| 8 Wochen | 8,0 | 3,4 |
| 9 Wochen | 8,0 | 3,4 |
| 10 Wochen | 8,0 | 3,4 |

## Patentansprüche

1. Triamin-trioxide der allgemeinen Formel

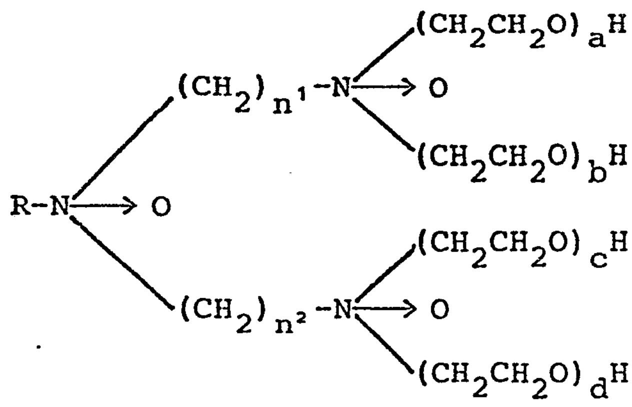

(I),

worin R einen gesättigten oder einen olefinisch ungesättigten Kohlenwasserstoffrest mit 1 bis 3 Doppelbindungen und von 8 bis 22 C-Atomen bedeutet, $n^1$ und $n^2$ eine ganze Zahl von 2 bis 3, wobei $n^1$ und $n^2$ gleich oder verschieden sein können, darstellt, sowie a, b, c und d, gleich oder verschieden, jeweils eine Zahl von 1 bis 5 ist, wobei die Summe a + b + c + d höchstens 10 betragen soll.

2. Verfahren zur Herstellung von Triamin-trioxiden gemäß Anspruch 1, bei dem zunächst ein primäres Amin der Formel $RNH_2$ (II) mit 2 mol mindestens eines reaktionsfähigen Nitrils von 2 bis 3 C-Atomen zu einer Verbindung der Formel

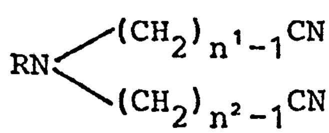

(III)

umgesetzt, in Gegenwart von Wasserstoff zu einer Verbindung der Formel

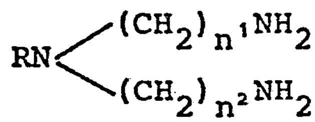 (IV)

reduziert und mit Ethylenoxid zu einer Verbindung der Formel

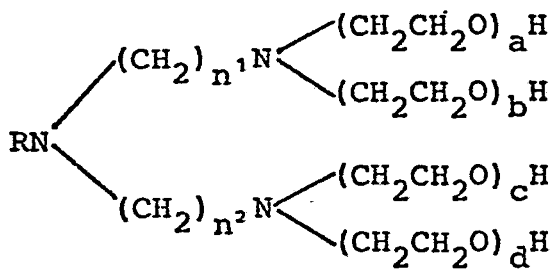 (V)

kondensiert wird, dadurch gekennzeichnet, daß diese Verbindung der Formel (V) in wäßriger Lösung mit Wasserstoffperoxid oxidiert wird.

3. Technisches Reinigungsmittel, enthaltend Wasser als flüssigen Träger, gegebenenfalls mindestens ein Tensid aus der Gruppe der anionischen, kationischen, nicht-ionischen oder amphoteren Tenside sowie gegebenenfalls reinigungsverstärkende Zusätze und übliche Hilfsstoffe, gekennzeichnet durch einen Gehalt an einem Triamin-trioxid der Formel (I) gemäß Anspruch 1.

4. Kosmetisches Reinigungsmittel, enthaltend Wasser als flüssigen Träger, gegebenenfalls mindestens ein Tensid aus der Gruppe der anionischen, kationischen, nicht-ionischen oder amphoteren Tenside, gegebenenfalls übliche kosmetische Zusätze und Hilfsstoffe, gekennzeichnet durch einen Gehalt an einem Triamin-trioxid der Formel (I) gemäß Anspruch 1.

<u>Patentansprüche für Österreich:</u>

1. Verfahren zur Herstellung von Triamin-trioxiden der allgemeinen Formel

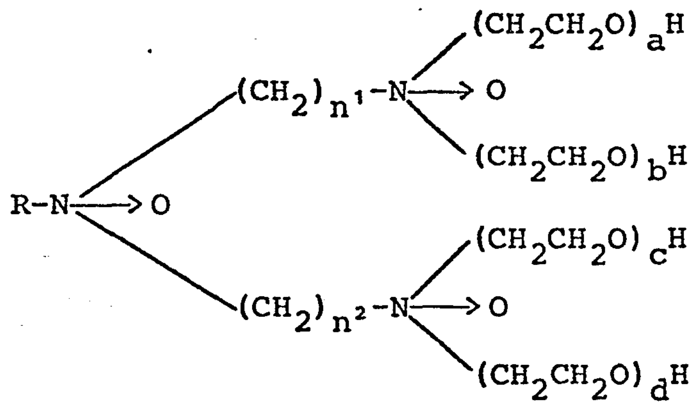

(I),

worin R einen gesättigten oder einen olefinisch ungesättigten Kohlenwasserstoffrest mit 1 bis 3 Doppelbindungen und von 8 bis 22 C-Atomen bedeutet, $n^1$ und $n^2$ eine ganze Zahl von 2 bis 3, wobei $n^1$ und $n^2$ gleich oder verschieden sein können, darstellt, sowie a, b, c und d, gleich oder verschieden, jeweils eine Zahl von 1 bis 5 ist, wobei die Summe a + b + c + d höchstens 10 betragen soll, bei dem zunächst ein primäres Amin der Formel $RNH_2$ (II) mit 2 mol mindestens eines reaktionsfähigen Nitrils von 2 bis 3 C-Atomen zu einer Verbindung der Formel

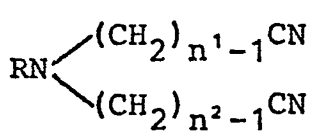

(III)

umgesetzt, in Gegenwart von Wasserstoff zu einer Verbindung der Formel

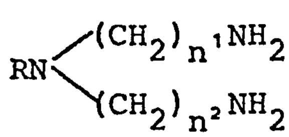

(IV)

0082299
HOE 81/F 928

reduziert und mit Ethylenoxid zu einer Verbindung der Formel

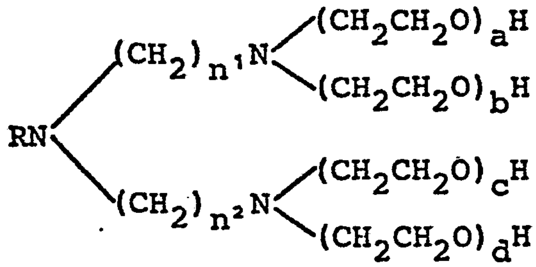

(V)

kondensiert wird, dadurch gekennzeichnet, daß diese Verbindung der Formel (V) in wäßriger Lösung mit Wasserstoffperoxid oxidiert und gegebenenfalls in Substanz isoliert wird.

2. Technisches Reinigungsmittel, enthaltend Wasser als flüssigen Träger, mindestens ein Tensid aus der Gruppe der anionischen, kationischen, nicht-ionischen oder amphoteren Tenside sowie gegebenenfalls reinigungsverstärkende Zusätze und übliche Hilfsstoffe, gekennzeichnet durch einen Gehalt an einem Triamin-trioxid der Formel (I) gemäß Anspruch 1.

3. Kosmetisches Reinigungsmittel, enthaltend Wasser als flüssigen Träger, mindestens ein Tensid aus der Gruppe der anionischen, kationischen, nicht-ionischen oder amphoteren Tenside sowie gegebenenfalls übliche kosmetische Zusätze und Hilfsstoffe, gekennzeichnet durch einen Gehalt an einem Triamin-trioxid der Formel (I) gemäß Anspruch 1.

Europäisches Patentamt

# EUROPÄISCHER RECHERCHENBERICHT

0082299

Nummer der Anmeldung

EP 82 11 0488

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| | --- | | C 07 C 135/02<br>C 11 D 1/75 |
| A | GB-A-1 007 343 (PROCTER & GAMBLE)<br>* Beispiele * | | |
| | --- | | |
| A | GB-A-1 064 188 (ARMOUR AND CO.)<br>* Beipiele II, III * | | |
| | ----- | | |
| | | | **RECHERCHIERTE SACHGEBIETE (Int. Cl. 3)** |
| | | | C 07 C 135/02<br>C 11 D 1/00 |

Der vorliegende Recherchenbericht wurde für alle Patentansprüche erstellt.

| Recherchenort | Abschlußdatum der Recherche | Prüfer |
|---|---|---|
| BERLIN | 10-02-1983 | BREW C.H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82